# EUROPÄISCHE PATENTANMELDUNG

(11) **EP 1 897 887 A1**
(43) Veröffentlichungstag der Anmeldung: **12.03.2008**
(21) Anmeldenummer: 06018847.1
(22) Anmeldetag: 08.09.2006
(51) Int. Cl.: C07K 14/02, C12N 15/62, A61K 39/00, C12N 7/04

(54) **Split-Core-Partikel für die Präsentation von Fremdmolekülen, insbesondere für Impfstoffanwendungen und Verfahren zu deren Herstellung**

(71) Anmelder: Universitätsklinikum Freiburg, 79106 Freiburg (DE)
(72) Erfinder: Nassal, Michael, Prof. Dr., 79232 March (DE); Walker, Andreas, 79106 Freiburg (DE); Skamel, Claudia, Dr., 79108 Freiburg (DE)
(74) Vertreter: Keller, Günter

(57) **Zusammenfassung**

Offenbart werden Split-Core-Trägerstoffe, die als getrennte Polypeptide die Core N- und die Core C-Domäne des Core-Proteins von Hepatitis B und wenigstens ein Fremdmolekül, gegen das eine Immunantwort induziert werden soll, aufweisen, wobei das Fremdmolekül, insbesondere die heterologe Fremdaminosäuresequenz an den C-Terminus der Core N-Domäne oder an den N-Terminus der Core C-Domäne fusioniert sind und das Core-Protein capsid-like-particles bilden kann, sowie Verfahren zu deren Herstellung.

## Beschreibung

Gegenstand der vorliegenden Erfindung sind neue Träger für Impfstoffe, aber auch für andere Moleküle, auf der Grundlage des Core-Antigens des Hepatitis B-Virus. In das Core-Antigen des Hepatitis-Virus werden fremde Aminosäuresequenzen eingebaut, die von Krankheitserregern stammen können. Gegen diese Proteinsequenzen sollen durch den Impfstoff Antikörper gebildet werden, wobei schützende oder neutralisierende Antikörper bevorzugt sind. Durch die erfindungsgemäßen Partikel kann auch die zelluläre Immunantwort (T-Zellen) stimuliert werden. Das Core-Protein von Hepatitis B hat die Eigenschaft, dass sich mehrere Kopien zu sogenannten capsid-like particles zusammenfügen können. Diese capsid-like particles (CLP) eignen sich besonders für die Herstellung von Impfstoffen, da sie das Immunsystem stimulieren und so zu einer gesteigerten Antikörperproduktion führen. Der Ausdruck "Impfstoff' ist in der vorliegenden Anmeldung bevorzugt als Trägersystem zu verstehen, das sowohl humorale wie auch zelluläre Immunantworten hervorrufen kann.

Kapside von Hepatitis B Viren sind ikosahedral symmetrische Nanopartikel (Durchmesser ca. 30 nm), die aus 180 bzw. 240 Kopien des viralen Core-Proteins bestehen. Das Core-Protein wird als HBcAg bezeichnet. Sie können als partikulärer Träger für Fremdmoleküle dienen; die bevorzugte Anwendung ist die als immunverstärkender Antigen-Träger für Impfstoffe. Durch geeignete Modifikation des Core-Proteins können die Fremdmoleküle auf der Partikel-Oberfläche (bei Bedarf auch im Inneren) präsentiert werden. Für die Oberflächenexposition ist die Verknüpfung des Fremdmoleküls mit zentral in der AS-Sequenz des Core-Proteins gelegenen AS-Resten (Bereich der AS ca. 73-94) optimal, welche das immundominante B-Zellepitop "c/e1" enthalten und in der 3D-Struktur am stärksten auf der Partikeloberfläche exponiert sind (AS bedeutet Aminosäure).

Die WO 01/77158 beschreibt Hepatitis B-Core-Antigen-Fusionsproteine, wobei heterologe Epitope bevorzugt in den Bereich zwischen den Positionen 61 und 90 eingesetzt werden. Die US 2003/0198649 beschreibt Hepatitis B-Virus Core-Antigen-Partikel, bei der die Immunogene über Ligandenstrukturen mit dem Hepatitis B-Virus (HBV) Core-Protein verbunden sind. Kratz et al. [PNAS (1999), 1915-1920] beschreiben die Präsentation von GFP (Green Fluorescent Protein) auf der Oberfläche des Coreproteins von HBV. Dabei wurden die Aminosäuren 79 und 80 des Core-Proteins durch die 238 Aminosäure lange Sequenz von GFP ersetzt.

Nassal et al. [Eu.J.Immunol. (2005), S. 655-665] beschreiben ein Fusionsprodukt des gesamten OspA-Proteins von Borrelia burgdorferi und des Hepatitis B-Virus-Kapsidproteins. Auch hier wurden die Aminosäuren 79 und 80 durch die Aminosäuren 18 bis 273 von OspA ersetzt, wobei allerdings zwischen Core-Protein und OspA Linkersequenzen eingebaut wurden. Skamel et al. (Journal of Biological Chemistry, 2006, Seiten 17474-17481) beschreiben die Präsentation des kompletten OspC-Proteins von Borrelia burgdorferi mit Hilfe von Hepatitis B-Virus-Kapsid-ähnlichen Partikeln. Bei den aus dem Stand der Technik bekannten Lösungen hat sich aber herausgestellt, dass die veränderten Coreproteine nicht mehr in gewünschter Weise zur Bildung von Kapsid-ähnlichen Partikeln (CLPs) neigen und, dass daher die Immunantwort nicht mehr zufriedenstellend stimuliert wird.

Bei einer genetischen Insertionen von Fremdmolekülen auf Peptid- bzw. Proteinbasis in das Core-Protein bedeutet dies, dass die Fremdsequenz sowohl über ihren N- wie ihren C-Terminus mit dem Core-Protein verbunden sind. Es wurde nun gefunden, dass die beidseitige Verknüpfung die Art und Auswahl geeigneter Fremdsequenzen drastisch einschränkt.

Das hier beschriebene "split-Core-System" hebt diese Einschränkungen auf, indem das Core-Protein in zwei separaten Teilen erzeugt wird, die sich überraschenderweise spontan zusammenfinden und vergleichbar der kontinuierlichen Proteinkette Kapsidpartikel bilden. Fremdmoleküle können entweder an das N-terminale ("coreN") oder das C-terminale ("coreC") Fragment fusioniert werden und sind dadurch nur noch über *ein* Ende mit dem Trägerprotein verknüpft. Die strukturellen Einschränkungen der beidseitigen Verknüpfung bei der Insertion von Fremdsequenzen in die kontinuierliche Peptidkette des Core-Proteins fallen damit weitestgehend weg.

Das erfindungsgemäße Split-Core-System erlaubt daher:
(i) Die Präsentation von Fremdmolekülen, die aufgrund ihrer Größe und/oder Struktur im konventionellen kontinuierlichen Core-Protein-Kontext nicht, oder nur sehr schlecht, präsentiert werden können;
(ii) Die Präsentation von heterodimeren Fremdproteinen;
(iii) Die Präsentation von interaktionsfähigen Fremdmolekülen in einer flexiblen, gut zugänglichen Form, welche die Interaktion mit den gewünschten Partnermolekülen wesentlich erleichtert;
(iv) Die Präsentation zusätzlicher Fremdmoleküle über die im split-Core, nicht aber im herkömmlichen kontinuierlichen Core-System, vorhandenen zusätzlichen oberflächenexponierten N- und C-Termini.

Hepatitis B Viren (HBVs) sind umhüllte Viren. Das innere Nukleokapsid wird auch als Corepartikel ("core") bezeichnet; serologisch ist es als Hepatitis B core antigen (HBcAg) definiert. Cores bestehen aus 180 bzw. 240 Kopien des 183-185 AS langen Core-Proteins (abhängig vom HBV Subtyp). Das Core-Protein kann heterolog (in Bakterien, Hefe, eukaryonten Zellen) exprimiert werden; dabei bilden sich spontan "capsid-like particles" (=CLPs). Da sie weder das Virusgenom noch die äußere Hülle enthalten, sind sie nicht infektiös. Solche CLPs lassen sich auch von den näher verwandten Säuger-HBVs (z.B. Waldmurmeltier = woodchuck; Erdhörnchen = grounds squirrel; und weitere) sowie den entfernter verwandten Vogel-HBVs (z.B. Ente = duck; Reiher = heron; und weitere) erhalten. Grundsätzlich können erfindungsgemäß alle HBV Sequenzen eingesetzt werden, bevorzugt sind allerdings die Sequenzen von HBVs, die Menschen infizieren können.

Die Aminosäuresequenz bzw. Nucleotidsequenz des HBV-Core-Proteins ist bekannt [Galibert et al., Nature (1979), S. 646-650; Nassal, Gene (1988), Seiten 279-294 oder WO 01/77158], auf diese Veröffentlichungen wird Bezug genommen. Bevorzugt eingesetzt wird die Core-Protein-Sequenz des Subtyps ayw, es können aber auch Varianten, Modifikationen der HBV-Sequenz ebenso verwendet werden, wie die Sequenz von anderen Säuger-HBVs oder Vogel-HBVs. Die Sequenzen sind in den öffentlich zugänglichen Genbanken gespeichert.

Natürliches HBcAg wie auch rekombinante HBV CLPs sind hochimmunogen (T-Zellunabhängig und T-Zell-abhängiges Antigen). Wesentlich dafür ist die symmetrische, multimere partikuläre Struktur des Corepartikels. Diese lässt sich in konventioneller "negative stain" Elektronenmikroskopie und auch Kryo-Elektronenmikroskopie darstellen. Biochemisch lässt sich die partikuläre Natur nachweisen durch Sedimentation in Sucrosegradienten (Sedimentationskoeffizient je nach Variante 60 - 80 S; zum Vergleich: lösliche monomere Proteine ca. 1-5 S; eukaryonte ribosomale Untereinheiten 40S+ 60S). Außerdem haben die Partikel ein distinktes Laufverhalten bei der nativen Gelelektrophorese in Agarosegelen, das sich von den nicht-partikulärer Formen unterscheidet. Dadurch ist feststellbar, ob sich CLP bilden können, oder ob die Proteine ohne die CLP-Struktur vorliegen.

Das Core-Protein weist 183 oder 185 Aminosäuren auf und besteht aus zwei Domänen: die ersten ca. 140 AS sind notwendig und hinreichend für das Assembly zu CLPs ("Assemblydomäne"); die C-terminale Region ist reich an basischen AS und bindet Nukleinsäuren. Eine C-terminal verkürzte Core-Protein-Variante (AS1-149) wurde in den ursprünglichen E. coli Expressionssystemen wesentlich besser exprimiert als das Volllängenprotein. CLPs aus Core-Protein 1-149, d.h. ohne Nukleinsäure-Bindungsdomäne wurden daher für mittel- und hochauflösende EM-Strukturuntersuchungen benutzt. So konnte die Kristallstruktur der CLPs und des Proteins bestimmt werden. Wie biochemisch vorhergesagt, bildet das Core-Protein sehr stabile, symmetrische Dimere, die sich zu CLPs aus 90 oder 120 Dimeren zusammenlagern.

Die Corepartikel besitzen auffällige "spikes"; jedes Dimer bildet einen spike, der strukturell aus einem 4-Helixbündel besteht, zu dem jedes Monomer 2 lange, zentral in der AS-Sequenz gelegene a-Helices beiträgt. Die spike-Spitzen umfassen die Aminosäuren (AS) in der Region von AS 74-85. Diese Region trägt auch das immundominante B-Zell-Epitop des HBcAg (c/e1-Epitop), seine besonders hohe Immunogenität erklärt sich sich aus der Oberflächen-Exposition dieser Reste.

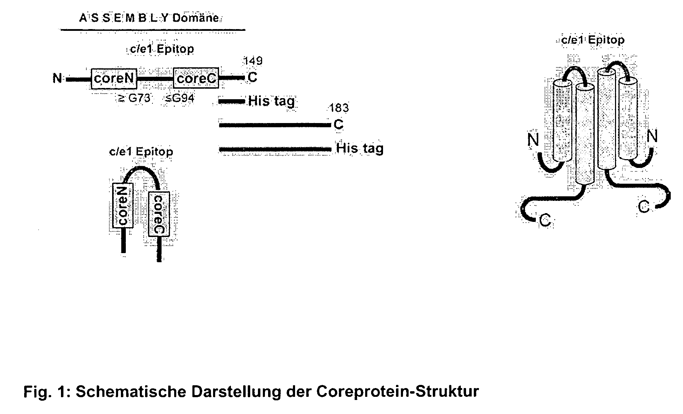

Figur 1 zeigt die Core-Protein-Struktur schematisch. Auf der linken Seite der Figur 1 ist die Primärsequenz des HBV-Core-Proteins dargestellt. Das Gesamtprotein besteht aus 183 AS (in einigen HBV Subtypen 185 AS); nur die N-terminalen ca. 140 AS werden zur CLP-Bildung benötigt (Assembly-Domäne); eine bevorzugte C-terminale AS-Position ist 149; an diese und jede folgende AS, bis AS183, kann ein His-tag fusioniert werden, ohne die CLP zu stören. Das c/e1-Epitop liegt im Bereich der AS 78 - 83, und bildet einen Teil des Loops zwischen den zentralen α-Helices (links unten). Die Helices selbst können verkürzt werden; minimal benötigt werden wahrscheinlich in coreN die AS-Reste bis Pos. Gly73, in coreC die Reste ab AS Gly94. Die rechte Seite der Fig. 1 zeigt eine schematische Darstellung eines Core-Protein-Dimers. Nur die beiden zentralen Helices pro Monomer sind gezeigt. Das c/e1 Epitop ist auf der Partikeloberfläche.

Für eine erfolgreiche Partikelbildung muss die Core AS-Sequenz mindestens Pos. 140 enthalten, vorzugsweise mindestens Pos. 149, kann sich aber auch weiter erstrecken bis zur C-terminalen AS 183 bzw. 185, je nach HBV Subtyp. Die AS-Sequenz nach Pos. 140, besser 149, kann durch Fremdsequenzen ersetzt werden, diese Fremdsequenzen befinden sich dann üblicherweise im Inneren der CLPs. Gegen solche Fremdaminosäuren werden nicht in erster Linie Antikörper gebildet, sie können aber andere Funktionen erfüllen.

Aufgrund der außergewöhnlich hohen Immunogenität des HBcAg sind rekombinante HBV CLPs attraktiv für die Verwendung als Immunogenitäts-steigernder Träger ("carrier") für Fremdantigene.

Ein alternativer Lösungsweg, der vorliegend nicht verfolgt wird, ist die *chemische* Kopplung von Fremdmolekülen an vorgeformte CLPs. Bei der vorliegenden Erfindung liegt eine *genetische Fusion* von Fremd-AS-Sequenzen an das Core-Protein vor. Hierbei stellte sich die Region des c/e1-Epitops noch vor der röntgenkristallographischen Strukturaufklärung anhand empirischer Versuche als bestgeeignet für die Induktion potenter B-Zellantworten heraus. Anhand der Struktur ist heute klar, dass solche Fusionen Insertionen in die Schleife ("loop") zwischen den zentralen Helices darstellen. Da dieser loop per se keine strukturgebende Funktion hat, kann die 3D-Struktur des Core-Proteins bei Insertion von Fremdsequenzen zumindest grundsätzlich erhalten bleiben. Abhängig von der inserierten Fremdsequenz können einige derartige Fusionsproteine weiterhin reguläre CLPs bilden, jedoch ist dies nicht immer gewährleistet, weil die Fremdaminosäuresequenz die Formung der Struktur des Fusionsproteins negativ beeinträchtigen kann. Die partikuläre Struktur ihrerseits ist essentiell für hohe Immunogenität.

Es wurden schon eine Vielzahl von Fremdsequenzen in das Core-Protein inseriert, z.T. auch die erhöhte Immunogenität experimentell nachgewiesen [Ulrich et al., Adv. Virus Res. (1998), S. 141-182]. Da viele Versuche zur Insertion von längeren Fremdsequenzen mit mehr als ca. 40 AS aber scheiterten (keine Partikelbildung mehr), nahm man an, es gebe eine natürlich begrenzte Aufnahme-Kapazität für Fremdsequenzen für bis zu 40 AS bzw. in einem speziellen Fall bis zu 120 AS.

Es wurde gefunden, daß Fremdaminosäuresequenzen, die am C- und N-Terminus mit dem Core-Protein fusioniert sind, nur dann Partikel ausbilden, wenn mindestens zwei essentielle Kriterien erfüllt sind:
(i) Die 3D-Struktur des Fremdproteins muss so geartet sein, dass sein N- und C-Terminus zur räumlichen Lage der Verknüpfungspunkte im Core-Protein (C-Terminus des N-terminalen Coreanteils, d.h. Core-AS ca. 1- bis ca. 78 [coreN]; N-Terminus des C-terminalen Coreanteils, d.h. Core-AS ca. 80 bis 149 oder 183 [coreN]) passen
(ii) falls das Fremdprotein selbst homomere Interaktionen eingeht (Dimere, Trimere, etc) muss die Struktur dieser Homo-Oligomere ebenfalls zur Struktur der beiden Core-Protein-Anteile passen

Gut geeignet für die Präsentation in vorbekannten Fusionsproteinen sind daher Fremdproteine, deren N- und C-Terminus in der nativen 3D-Struktur nahe beieinander liegen. GFP erfüllt beide dieser Voraussetzungen, viele andere Proteine aber nicht; ein Beispiel hierfür ist das outer surface protein A (OspA) des Lyme-Borreliose Erreger *Borrelia burgdorferi,* dessen N- und C-Terminus auf entgegengesetzten Seiten der langgestreckten 3D-Struktur liegen.

Die Insertion von OspA oder ähnlich ungünstig strukturierten anderen Proteinen führt zu Spannungen im Fusionsprotein. Entweder bleibt das Fremdprotein korrekt gefaltet und verhindert die Annäherung der beiden Core-Proteinanteile, so dass deren Faltung und nachfolgende Dimerisierung und Partikelbildung verhindert wird; oder die Faltung des Core-Protein-Anteils beeinträchtigt die Faltung des Fremdproteins, so dass dieses entweder nicht mehr nativ vorliegt (> veränderte Antigenität), oder - falls massiv missgefaltet - zur Aggregation führt.

Im Fall von OspA konnte dieses Problem nur partiell durch Benutzung sehr langer Verbindungssequenzen ("Linker") umgangen werden. Gerade für Vakzine-Anwendungen stellen diese Linkersequenzen ein potentielles Problem dar, da sie unerwünschte eigene Antigenität besitzen könnten, die zu schädlichen Kreuzreaktionen, bspw. mit körpereigenen Antigenen, führen können. Außerdem waren entsprechende Protein-Präparationen nur sehr begrenzt zur Bildung regulärer CLPs in der Lage.

Aufgrund der dimeren Struktur des Coreprotein-Trägers und der geometrisch begrenzten Oberfläche der Trägerpartikel könnte bei sehr großen oder stark von einer globulären Struktur abweichenden Fremdproteinen zusätzlich *generelle* sterische Hinderung ein Problem darstellen (der auf der "Kugeloberfläche" der CLPs verfügbare Raum ist begrenzt). Die Maximalgröße des Fremdproteins hängt von mehreren Faktoren ab. Mit dem erfindungsgemäßen Verfahren gelang schon die Präsentation eines ca. 320 AS umfassenden Fremdproteins (CSP).

Gegenstand der vorliegenden Erfindung ist daher ein Split-Core-Impfstoff, der als getrennte Polypeptide die Core N- und die Core C-Domäne des Core-Proteins von Hepatitis B und wenigstens eine Fremdaminosäuresequenz, gegen die eine humorale sowie gegebenenfalls zelluläre Immunantwort hervorgerufen werden soll, aufweist, wobei die Fremdaminosäuresequenz an den C-Terminus der Core N-Domäne oder an den N-Terminus der Core C-Domäne fusioniert ist und das Core-Protein capsid-like-particles binden kann. Bevorzugt werden durch das Trägersystem neutralisierende Antikörper hervorgerufen.

Wesentlich bei dem erfindungsgemäßen Split-Core-Impfstoff ist, dass das Core-Protein am c/e1 Epitop, also etwa zwischen den Aminosäuren 73 und 94 unterbrochen, also getrennt ist. Die Fremdaminosäure kann nun an das C-terminale Ende des Core N-Bereichs anfusioniert sein. In diesem Falle beginnt das N-terminale Ende des Core C-Teiles mit derjenigen Aminosäure, bei der das Core N-Protein getrennt wurde. Es ist auch möglich, dass ein Teil des c/e1 Epitops deletiert ist, dass also eine oder mehrere Aminosäuren aus dem Bereich zwischen Aminosäure 73 und 94 entfernt wurden. Alternativ hierzu ist möglich, dass die Fremdaminosäuresequenz, gegen die Antikörper gebildet werden sollen, an das N-terminale Ende des Core C-Bereichs anfusioniert ist. In diesem Falle endet der C-terminale Bereich von Core N an der Aminosäure, bei der das Core-Protein unterbrochen wurde. Ein wesentlicher Effekt, den der Split-Core-Impfstoff beibehalten muß, ist, dass die Split-Core-Impfstoffpartikel, die anfusionierte Fremdaminosäuresequenzen tragen, dennoch die corecapsid-like particles bilden können. Dies wird entweder im Saccharose-Gradienten oder in der nativen Agarose-Gelelektrophorese überprüft. Auch eine Überprüfung mit Hilfe des Elektronenmikroskops ist möglich.

Bei der Fremdaminosäuresequenz, gegen die Antikörper gebildet werden sollen, handelt es sich bevorzugt um Sequenzen, die von Oberflächenstrukturen von Mikroorganismen herstammen. Derartige Mikroorganismen rufen üblicherweise Erkrankungen beim Menschen hervor. Wenn gegen diese Strukturen neutralisierende Antikörper gebildet werden können, führt dies dazu, dass die Immunabwehr eingedrungene Mikroorganismen sehr schnell beseitigen kann. Ein Bakterium, bei dem die Impfstoffentwicklung schon relativ weit fortgeschritten ist, ist Borrelia burgdorferi, der Erreger der Lyme Disease. Bevorzugt eingesetzt werden im Rahmen der Erfindung als Fremdaminosäuresequenzen die Oberflächenproteine OspA und OspC von Borrelia burgdorferi. Bei den Fremdaminosäuresequenzen kann es sich aber auch um andere Sequenzen handeln, die von pathogenen Organismen herstammen. Ein derartiges Beispiel ist der Malaria-Erreger Plasmodium falciparum.

Mit dem vorliegenden Split-Core-Impfstoff können aber nicht nur Antikörper gegen körperfremde Aminosäuresequenzen erzeugt werden, sondern auch Antikörper gegen unerwünschte körpereigene Strukturen, wie beispielsweise Tumormarker. Tumorzellen exprimieren häufig andere Oberflächenmarker als gesunde Zellen. Wenn also derartige körpereigene Aminosäuresequenzen im Split-Core-Impfstoff präsentiert werden, wird die Bildung einer Immunantwort angeregt, so dass der Körper in verstärktem Ausmaß Antikörper gegen die Tumorzellen bildet. Durch diese Antikörper können die Tumorzellen leicht von der Immunabwehr erkannt und schließlich eliminiert werden. Durch das erfindungsgemäße Trägersystem kann aber auch die zelluläre Immunantwort angeregt werden, was zu einer verbesserten Wirksamkeit führen kann.

Gegenstand der vorliegenden Anmeldung ist auch ein Verfahren zur Herstellung eines Impfstoffes gegen eine heterologe Proteinsequenz. Der Split-Core-Impfstoff besteht aus zwei Teilen des Core-Antigens eines Hepatitis B-Virus, wobei entweder an die Core N-Region die heterologe Proteinsequenz anfusioniert ist, oder an das C-terminale Ende der heterologen Proteinsequenz das Core C-Protein anfusioniert ist. Diejenige Position in der Aminosäurekette, an der die beiden Domänen voneinander getrennt sind, befindet sich zwischen Position 73 und Position 94. Einzelne Aminosäuren dieser Aminosäureregion, die das c/e1 Epitop darstellen, können deletiert sein.

Das erfindungsgemäße Verfahren kann grundsätzlich auf zwei verschiedene Arten durchgeführt werden. Wenn die beiden Teile des Split-Core-Antigens als nur eine Polypeptidkette exprimiert werden, wird erfindungsgemäß eine Erkennungssequenz für eine Protease an der vorgesehenen Position eingebaut. Dadurch kann das Polypeptid nach Expression durch die Protease in zwei definierte Teile gespalten werden. Bei dieser Ausführungsform ist es auch möglich, diejenige Protease, die an der vorgesehen Schnittstelle schneidet, mitzuexprimieren. Die hierfür kodierende Gensequenz kann sich entweder auf demselben Vektor oder auf einem separaten Vektor befinden.

In einer anderen, bevorzugten Ausführungsform werden die beiden oben erwähnten Polypeptide aber mit einem speziellen Vektor exprimiert. Bevorzugt eingesetzt wird ein sogenannter bicistronischer Vektor. Dies bedeutet, dass der eine Teil des Split-Core-Impfstoffes als Polypeptidkette exprimiert wird und, dass sich am C-Terminus ein Stopkodon befindet. Kurz danach können aber die Ribosomen wieder ansetzen und das zweite Polypeptid exprimieren. Dadurch wird gewährleistet, dass sich die beiden Teile des Split-Core-Impfstoffes in etwa gleicher Menge bilden und es müssen keine Vorsichtsmaßnahmen getroffen werden, damit der Wirtsorganismus nicht einen der Vektoren verliert, was dazu führen würde, dass nur noch ein Teil des Split-Core-Impfstoffes bereitgestellt wird.

Der Ausdruck "Fusionsprotein" oder "anfusioniert" bedeutet, dass zwei unterschiedliche Proteine oder Polypeptide über eine Peptidbindung miteinander verbunden sind. Solche Fusionsproteine entstehen durch Expression der dafür kodierenden, hintereinander geschalteten Nucleinsäuresequenzen.

Es wurde im Rahmen der Erfindung gefunden, daß sich als proteinchemisches Hauptproblem für eine breitere Verwendbarkeit des HBV-CLP Trägersystems (spezifisch als Impfstoff-Carrier, generell für die Präsentation von Fremdmolekülen) die beidseitige Verknüpfung des inserierten Fremdproteins über N- und C-Terminus darstellt. Läßt sich *eine* der beiden kovalenten Bindungen (auf der *N-oder* der C-terminalen Seite des Inserts) lösen, und kommt es trotzdem zur Partikelbildung, weil sich die getrennten Core-Protein-Anteile ("split-core") finden und korrekt falten, erhöht sich die Anzahl und Art der inserierbaren Fremdproteine drastisch. Dies ist ein wesentlicher Aspekt der vorliegenden Erfindung.

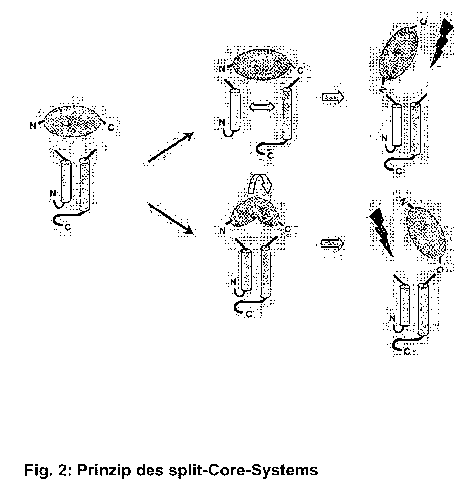

Figur 2 zeigt schematisch, dass die Insertion eines Fremdproteins mit ungünstiger 3D-Struktur (z.B. OspA) die Bildung der Partikel-bildungskompetenten Struktur des Core-Protein-Trägers verhindern kann (oberer Weg); alternativ kann die Ausbildung der korrekten 3D-Strukturs des Core-Protein-Trägers die Formation der nativen 3D-Struktur des Fremdproteins verhindern (unterer Weg). Dies würde die Bindung der gewünschten Antikörper ungünstig beeinflussen. Diese sterische Problematik wird aufgehoben, wenn eine der beiden kovalenten Verknüpfungen zwischen Fremdprotein-Insert und Träger (auf der C-terminalen oder der N-terminalen Insert-Seite) gelöst wird (Pfeil). Eine Möglichkeit der Realisierung ist die nachträgliche Spaltung eines kontinuierlichen Fusionsproteins; dies erfordert die zusätzliche Einführung einer Schnittstelle für eine spezifische Protease. Bevorzugt werden daher die beiden Fragmente von vornherein als separate Entitäten exprimiert.

Durch die erfindungsgemäße Lösung werden noch weitere Konsequenzen bewirkt:
(i) da das c/e1 Epitop auf der Partikeloberfläche liegt, entstehen auf ebendieser Oberfläche neue N-und C-Termini, die weiter derivatisiert werden können. Z.B. kann an das coreN-Fragment ein Fremdmolekül X, und an das coreC-Fragment ein Fremdmolekül Y fusioniert werden. Eine mögliche Anwendung ist die Präsentation von heterodimeren Fremdmolekülen. Hierbei muß allerdings beachtet werden, dass keine sterische Hinderung der beiden anfusionierten Teile auftreten darf.
(ii) spezifische Teilregionen einer inserierten Fremdsequenz, z.B. ein besonders wichtiges Epitop können je nach Verknüpfung über N- oder C-Terminus zur Partikeloberfläche hin, oder von ihr weg, orientiert werden. Ein Beispiel ist das sog. LA2-Epitop von B. burgdorferi OspA, das nachgewiesenermaßen im C-terminalen Bereich liegt. Antikörper, die dieses Epitop erkennen, sind neutralisierend.

Dies ist auch wichtig für die Insertion von Fremdsequenzen, die ihrerseits weitere Interaktions-Oberflächen darbieten (für die Anheftung dritter Moleküle; siehe Beispiele). Da nur einseitig verknüpft, wird der interaktionsfähigen Fremdsequenz keine artifizielle 3D-Struktur aufgezwungen; stattdessen ist sie, je nach Art, flexibel oder kann ihre korrekte 3D-Struktur ungestört einnehmen. Damit werden Interaktionen mit dem Interaktionspartner wesentlich erleichtert.

### Beispiel 1

### Wildtyp-Core-Protein mit inserierter TEV-Protease Erkennungssequenz

In das c/e1 Epitop von wt-Coreprotein 1-149 wurde eine Erkennungssequenz für die tobacco-etchvirus (TEV) Protease eingeführt (Ersatz der AS P79+A80 durch GGGGT-ENLYFQGT-GGGG; G-Reste als Linker, um Zugänglichkeit der Erkennungssequenz für die Protease zu gewährleisten). Das rekombinant exprimierte Protein formte Partikel. Diese wurden mit TEV Protease inkubiert, der Erfolg der Spaltungsreaktion durch SDS-PAGE überprüft. Der Protease-Verdau-Ansatz wurde anschließend durch einen Sucrosegradienten sedimentiert. Es kam zur praktisch vollständigen Spaltung an der erwarteten Position, da 2 nahezu gleich große Fragmente entstanden. Trotz Spaltung sedimentierte das Protein im Gradienten etwa an dieselbe Position wie ungespaltenes wt-Core-Protein 1-149. Vergleichbare Ergebnisse wurden mit einem Fusionsprotein auf Basis des Volllängen-Core-Proteins 1-183 erhalten.

### Beispiel 2

### Core-Protein-Varianten mit größeren inserierten Fremdsequenzen

Als Beispiel für ein Fusionsprotein mit moderat großer Insertion wurde in das c/e1-Epitop die Sequenz für ein artifizielles Peptid "ACID" (flankiert von Gly-reichen Linkern; gesamt 65 AS) inseriert, welches mit einem komplementären Peptid "BASE" interagieren kann [O'Shea et al., Curr.Biol. (1993), 658-667]; daran anschließend die TEV-protease-Erkennungssequenz

GGGGSGGGVEDGGGGSGGGGT-AQLEKELQALEKENAQLEWELQALEKELAQTG-
ENLYFQGTGGGG.

Das Fusionsprotein bildete CLPs. Diese wurden isoliert und wie oben mit TEV-Protease inkubiert. Auch hier kam es zu spezifischer Spaltung, die Partikel blieben aber intakt. In diesem Fall sind die TEV-Spaltfragmente unterschiedlich groß und daher im SDS-Gel direkt zu unterscheiden. Trotz Spaltung ko-sedimentieren die Fragmente in die CLP-spezifischen Gradienten-Fraktionen.

Dieses Beispiel zeigt, dass sich auch CLPs aus Core-Protein mit einem inserierten 65 AS Peptid (Linker + ACID + TEV-Schnittstelle) spezifisch spalten lassen, die partikuläre Struktur bleibt trotzdem erhalten.

### Beispiel 3

Als weiteres Beispiel ist ein entsprechender Versuch mit einem Core-Protein gezeigt, das die AS Sequenz 18-273 des Borrelia burgdorferi OspA Proteins enthält. Das entsprechende Protein ohne TEV-Spaltstelle ist in Nassal et al., 2005 beschrieben; nur ein Bruchteil davon bildete reguläre Partikel, was sich auch in einer breiten Verteilung im Sucrosegradienten gezeigt hatte. Der nicht geschnittene Impfstoff ist also als solcher nur bedingt zu gebrauchen. Da zudem sehr lange Extra-Linkersequenzen mit eigenem, unerwünschten Antigenpotential notwendig waren, um wenigstens einen kleinen Anteil der Präparation in CLP-Form zu erhalten, wäre dieser nicht geschnittene Impfstoff nicht im Menschen/medizinisch anwendbar.

Nach Einführung einer TEV-Spaltstelle wurde das Fusionsprotein wie in Beispiel 2 mit TEV-Protease behandelt, dann im Sucrosegradienten sedimentiert. Auch hier kam es zur spezifischen Spaltung, allerdings nicht vollständig (vermutlich durch sterische Hinderung via OspA schlechtere Zugänglichkeit der TEV-Schnittstelle); Zuordnung der Fragmente mittels monoklonaler Antikörper, die spezifisch den N-terminalen bzw. C-terminalen Coreanteil erkennen; der α-coreN Antikörper reagiert demzufolge mit dem ungespaltenen Fusionsprotein sowie mit dem N-terminalen Spaltfragment. Trotzdem zeigte das partiell gespaltene Material ein Partikel-typisches Laufverhalten.Diese Daten deuteten darauf hin, dass durch Öffnen einer der beiden Verbindungen zwischen inseriertem Fremdprotein und Coreträger die Partikelbildung deutlich verbessert werden konnte.

Das Beispiel belegt, dass sich auch ein Corefusionsprotein mit einer *großen* Fremdprotein-Insertion (255 AS OspA + Linker + TEV-Schnittstelle) spezifisch spalten läßt (wenn auch weniger effizient). Während das zuvor beschriebene kontinuierliche coreOspA Fusionsprotein sich breit über den Gradienten verteilt (Nassal et al., 2005), reichert sich das (partiell) gespaltene Fusionsprotein distinkt in partikeltypischen Gradientenfraktionen an. Dies ist ein Indiz für verbesserte Partikelbildungseffizienz durch die Spaltung.

In einem weiteren Versuch konnte gezeigt werden, dass (von einem kompatiblen Plasmid) ko-exprimierte TEV-Protease zur Spaltung von TEV-Schnittstellen tragenden Corefusions-Proteinen führt, teilweise effizienter als durch nachträgliche in vitro Spaltung möglich. Auch für die schon in den Bakterien gespaltenen Fusionsproteine konnte Partikelbildung via Sucrosegradienten-Sedimentation nachgewiesen werden.

Die Beispiele 1 bis 3 zeigten, dass *vorgeformte* CLPs nach Spaltung im Bereich des c/e1 Epitops intakt bleiben. Dies ist strukturell damit zu erklären, dass die Verbindungsschleife zwischen den spike-bildenden Helices keine *per se* strukturgebende Rolle hat, konnte so aber nicht erwartet werden. Dies führte zu der bevorzugteren Ausführungsform, bei der, statt nachträglicher Spaltung der kontinuierlichen Proteinkette die coreN und coreC-Teile direkt als separate Proteinfragmente exprimiert werden, wobei sie sich offensichtlich spontan zusammenfinden und Partikel bilden. Ein solcher Ansatz hat mindestens drei wesentliche Vorteile:
(i) Vereinfachung: der zusätzliche Spaltungsschritt mit TEV-(oder einer anderen) Protease ist nicht mehr notwendig
(ii) keine zusätzliche Peptidsequenz im Fusionsprotein für die spezifische Protease-Erkennung notwendig
(iii) eine - wenn auch nur partielle - Partikelbildung mit sterisch ungünstigem Fremdprotein wie OspA erforderte sehr lange Linkersequenzen; diese sind verzichtbar, wenn sterische Hinderungen durch die nicht mehr beidseitig notwendige Verknüpfung mit dem Core-Trägerprotein vermieden würden.

Die Punkte (ii) und (iii) sind besonders für Vakzineanwendungen bedeutsam, da jegliche zusätzliche Sequenz zu unvorhersehbaren immunologischen Konsequenzen führen kann (neue Epitope durch die Zusatzsequenzen, möglicherweise Kreuzreaktionen mit körpereigenen Epitopen)

### Beispiel 4

### Expressionkonstrukte zur annähernd, äquimolaren Expression von coreN und coreC Fragmenten

In der kontinuierlichen Peptidkette liegen die späteren Spaltprodukte zwangsläufig in äquimolaren Mengen vor. Bei separater Expression sollten die beiden Teile daher für eine effiziente Zusammenlagerung ebenfalls in annähernd äquimolaren Mengen entstehen. In ersten Versuchen wurden zwei separate, kompatible Plasmide zur Expression der beiden Trägerprotein-Teile eingesetzt, allerdings mit mäßigem Erfolg.

In bevorzugter Ausführungsform werden bicistronische Vektoren eingesetzt, die zudem den Vorteil bieten, dass auf Selektion mit einem weiteren Antibiotikum (für den Erhalt des ansonsten notwendigen zweiten Plasmids) verzichtet werden kann.

Typ1 Konstrukte enthalten hinter einem gemeinsamen Promotor (hier T7 Phagen RNA Polymerase Promotor) zwei Expressionskassetten mit jeweils einer vorgeschalteten Ribosomen-Bindungsstelle (ribosome binding site, RBS; "Shine-Dalgarno-Sequenz"). In den hier realiserten Vektoren wird die Translation des coreN Fragments durch ein artifizielles Stop-Codon nach AS Prolin 79 (P79) beendet, die Translationsinitiation des 2. Fragmentes durch ein artifizielles Start-Codon vor AS Serin81 (S81) ermöglicht. Eine solche Operonstruktur ist in Bakterien häufig zu finden ("polycistronische mRNAs"; Ribosomen können relativ unabhängig an jede RBS auf der mRNA binden und die Translation des 3' gelegenen Gens initiieren). In den realisierten Konstrukten ist die zweite RBS eine exakte Kopie der ersten RBS, die aus dem originalen pET-Vektoren stammt; andere RBS-Sequenzen sind mit Sicherheit ebenfalls möglich. Das ATG Startcodon des 2. Cistrons ist Teil einer Nde-Schnittstelle (CATATG) für das leichte Einklonieren von Fremdsequenzen.

**Typ2 Konstrukte** ("stop/start") enthalten keine separate zweite RBS; stattdessen überlappt mit dem Stopcodon des *upstream* Gens (NNN TGA) ein Inititionscodon für das *downstream* Gen (NNA TGA) in einem um 1 verschobenen Leseraster. In diesem Fall können Ribosomen nach Ablesen des ersten Gens direkt am ATG des zweiten Gens re-initiieren; eine solche Anordnung erlaubt die Koexpression zweier Gene; ähnliche Stop-Start Arrangements finden sich in einigen Bakteriophagen. Sie ist auch in einigen eukaryonten non-LTR Retrotransposons verwirklicht (Verbindung ORF1/ORF2). Anhand der bekannten Sequenzen z.B. im E. coli Trp Operon sollten andere Kombinationen von nahe benachbarten, oder überlappenden Stop-/Startcodons (z.B. TAA TG, TGA TG, etc.) ähnlich geeignet sein.

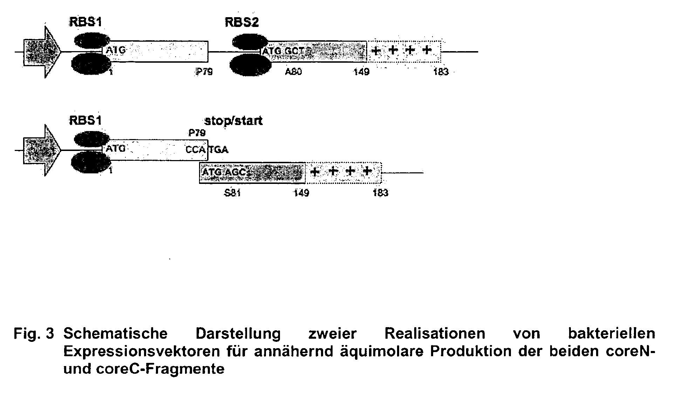

Bevorzugte Expressionsvektoren sind schematisch in Fig. 3 dargestellt.

Oben: Typ 1 Konstrukte. Ein Promotor, z.B. für T7 RNA Polymerase, führt zur Transkription einer bi-cistronischen mRNA (für effiziente Termination kann 3'-seitig eine Terminatorsequenz eingefügt sein). Das erste Cistron kodiert die HBV Coreprotein-AS-Sequenz 1-79. Eine vorgeschaltete Ribosomen-bindungsstelle (RBS1) ermöglicht die Translationsinitiation (kleine und große Ribosomen-Untereinheit als Ovale dargestellt). Am 3'-Ende des Codons für P79 folgt ein Stop-Codon. Am 5'-Ende des zweiten Cistrons liegt eine weitere RBS (RBS2) für die Translationsinitiation des coreC-Fragments. Dieses verfügt dazu über ein eigenes Initiationscodon (ATG). Im gezeigten Beispiel beginnt die coreC-Sequenz mit Ala80; sie kann sich bis AS-Position 140, besser 149, oder bis zum authentischen Ende bei Pos. 183 erstrecken. Die AS-Sequenz ab Pos. 140, besser 149, kann auch durch Fremdsequenzen ersetzt werden.

Unten: Typ2 Konstrukte. Diese enthalten nur eine RBS vor dem ersten Cistron. Die Translation des coreC-Fragments erfolgt durch Re-Initiation, indem das artifizielle Startcodon von coreC (ATG) mit dem Stop-Codon von coreN (TGA) wie gezeigt überlappt. Im Bespiel beginnt die coreC-Sequenz mit Ser81.

Die AS 77, 78 und 79 des Core-Proteins sind Glutamat (E; Codons: GAA, GAG), Aspartat (D; Codons: GAC oder GAT), AS 79 Prolin (P; Codons: CCN). In den realisierten Typ 1 Konstrukten wurde für die AS-Sequenz EDP die Nukleotid-Sequenz GAG GAT CCN gewählt, wodurch eine Schnittstelle für BamHl entsteht (GGATCC); durch diesen singulären BamHl Schnitt ist eine simple Einklonierung von unterschiedlichen coreN- und coreCkodierenden DNA-Fragementen möglich. Das Nucleotid N im Prolin Codon CCN ist nicht festgelegt; das Stop-Codon kann TAA, TAG, oder TGA sein.

In Typ 2 Konstrukten lautet die Sequenz bis zum Ende des ersten Gens G GAT CCa TGA. Auch hier ist eine BamH1 Schnittstelle (unterstrichen) für das Einklonieren unterschiedlicher coreN bzw. coreC kodierender DNA-Fragmente vorhanden. Für P79 muss in den realisierten Typ2-Konstrukten das Codon CCa sein, das Stop-Codon muss TGA (> CCa TGA) sein, um das Entstehen eines überlappenden ATG Initiationscodons für das zweite Gen zu erlauben. Das Codon für die erste AS des zweiten Gens nach dem ATG-Startcodon muss mit A beginnen. Alternative Stop/Start Nt-Folgen wären TGA TG oder TAA TG wie im E. coli Trp Operon zwischen TrpE und TrpD oder auch TrpB und TrpA [Das and Yanofsky (1989), Nucl. Acids Res., S. 9333-9340; Oppenheim and Yanofsky (1980), Genetics, S. 785-795].

In den realisierten Vektoren (pET28a2-xxx) beruht das Grundgerüst auf dem kommerziellen Vektor pET-28, bei dem jedoch das Kanamycin-Resistenzgen gegen dasjenige für Ampicillin-Resistenz ausgetauscht wurde. Mit Sicherheit sind jedoch andere Vektor-Grundgerüste ebenfalls anwendbar (verschiedene replication oris, verschiedene Resistenzgene, verschiedene Promotoren, verschiedene RBS). Essentiell ist die bicistronische Anordnung der Gene für coreN und coreC, entweder durch 2 separate RBS, oder durch Stop/Start Anordnung. In eigenen Versuchen liessen sich mehrere split-Core Proteine von Typ1 wie von Typ 2-Konstrukten exprimieren. Für Typ 2 (Stop/Start) Konstrukte war dies jedoch in einer geringeren Anzahl von Konstrukten der Fall. Ausserdem ist die mögliche Sequenzvariabilität bei Typ2-Konstrukten geringer (s.o.). Typ1 Konstrukte sind daher breiter anwendbar.

Als C-terminale AS des coreN-Anteils wurde P79 gewählt, da Prolin besonders Proteaseresistent ist und somit eine Degradation des Fragments minimiert wird. Aufgrund der bekannten 3D-Struktur sowie beschriebener Deletionsvarianten in dieser zentralen Region lässt sich aber annehmen, dass
(i) das Ende des coreN Fragments im Bereich der AS ca. ab Pos. 72 - 74, besonders Gly73
(ii) der Beginn des coreC Fragments im Bereich der AS ca. ab Pos. 78-86, möglicherweise bis Gly94 liegen könnte, ohne dass wesentlich andere Ergebnisse als mit den hier gewählten Grenzen (coreN Ende bei P79, coreC Start bei S81, mit vorgeschaltetem Start-ATG) erhalten würden.

Für die Expression in eukaryonten Zellen sind die beschriebenen Konstrukte aufgrund des unterschiedlichen Transkriptions- und Translations-Mechanismus so nicht anwendbar; statt des prokaryonten Promotors muß ein eukaryonter Promoter verwendet werden (z.B. CMV-IE und viele andere), sowie ein Poly-Adenylierungssignal (z.B. aus SV40 Virus, oder viele andere) nach dem Ende des/der offenenen Leseraster(s) eingeführt werden.

Eine annähernd gleiche Expression von coreN und coreC in Eukaryonten kann erreicht werden durch:
(i) Ko-Transfektion zweier Plasmide (je eines für coreN, eines für coreC);
(ii) Transfektion eines Plasmids mit 2 unabhängigen Expressions-Kassetten;
(iii) Transfektion eines Plasmids mit funktionell bicistronischer mRNA; z.B. könnte das zweite Gen durch eine vorgeschaltete "internal ribosome entry site" (IRES) exprimiert werden;
(iv) Möglicherweise ist auch ein Typ2-Vektor-artige Stop-/Start Anordnung in Eukaryonten funktionell.

### Beispiel 5

### Separate Expression von wt-coreN und coreC Fragmenten führt zur Bildung intakter CLPs

Mittels Expressionsvektoren auf Typ1-Basis (Fig. 3) wurde Wildtyp-Core-Protein in zwei Teilen in E. coli BL21 Zellen exprimiert, einmal auf Basis der AS 1-149, einmal AS 1-183. Beide erwarteten Fragmente wurden gebildet und lagerten sich zu intakten CLPs zusammen. Dies wurde mit Sucrose-Gradienten sowie nativ-Agarosegel-Elektrophorese nachgewiesen. Als direkter Beweis für die Bildung von CLPs wurde ein elektronenmikroskopischer Vergleich (negative staining) von Partikeln aus kontinuierlicher AS 1-183 Kette mit C-terminalem His6-tag vs. Partikeln aus dem entsprechenden split-Core-System durchgeführt. Bei dieser Auflösung waren keine Unterschiede detektierbar.

Wt-HBV-CLPs sind sehr robust und beständig gegen Harnstoff in molaren Konzentrationen; CLPs aus C-terminal trunkiertem Core-Protein 1-149 lassen sich durch 3-5 M Harnstoff in Dimere dissoziieren und durch Änderung der Pufferbedingungen (Entfernen des Harnstoffs, neutraler pH, erhöhte Salzkonzentration) in vitro wieder zu CLPs reassoziieren. Eine vergleichende Untersuchung zur Stabilität der CLPs aus split-Core zeigte eine ähnlich hohe Stabilität der split-Core CLPs. Dies zeigt die hohe Affinität der coreN und coreC-Anteile zueinander, und ist wichtig für die Verwendung als Trägersystem für Fremdmoleküle.

### Beispiel 6

### Separate Expression von Fremdprotein-fusionierten-coreN und wt-coreC Fragmenten, sowie vice versa, führt zur Bildung intakter CLPs

Zum Nachweis der grundsätzlichen Eignung des split-core-Systems für die CLP-Bildung und Präsentation von Fremdproteinen wurde als Modell GFP entweder an coreN oder an coreC fusioniert; für das coreN-Konstrukt wurde entweder ein Typ1 (zweite RBS) oder Typ2 (Stop/Start) Vektor benutzt. Alle Konstrukte führten zur Expression grün fluoreszierender Proteine, die laut Sedimentation in Sucrosegradienten Partikel bildeten.

Zum weiteren Nachweis der physikalischen Assoziation der jeweiligen coreN und coreC Fragmente wurden Aliquots der Gradientenfraktionen der Elektrophorese in nativen Agarosegelen unterzogen. Dabei bleiben Partikel erhalten, ebenso der GFP-Chromophor. In den Fraktionen fand sich sowohl bei GFP an coreN, wie GFP an coreC, eine distinkte, grün fluoreszierende Bande; nicht assembliertes GFP Fusionsprotein verblieb in den oberen Gradientenfraktionen, und zeigte ein anderes Laufverhalten sowie eine diffusere Verteilung (schnellere Diffusion im Gel aufgrund der wesentlich kleineren Struktur im Vergleich zum Partikel aus 180 oder 240 Untereinheiten). Sowohl für GFP an coreN wie an coreC ließ sich mit dem α-coreC Antikörper in der grün fluoreszierenden (dito GFP enthaltenden) Bande auch coreC nachweisen.

Dieses Beispiel zeigt zweierlei, nämlich
(i) Im split-core-System kann ein Fremdprotein von ca. 240 AS, hier GFP, sowohl an coreN wie an coreC fusioniert werden, ohne die CLP-Bildung zu stören.
(ii) Sowohl Typ1 wie Typ2 split-Core-Vektoren sind für die Ko-Expression von coreN und coreC mit anfusionierter Fremdsequenz geeignet.

### Beispiel 7

### CLP-Präsentation von medizinisch relevanten Fremdproteinen im splitcore-System, die konventionell nicht, oder nur sehr eingeschränkt, möglich ist

### Borrelia burgdorferi OspA

Wie eingangs dargelegt, führt die im bisherigen System erforderliche beidseitige Verknüpfung des inserierten Fremdproteins mit dem Trägerprotein zu starken topologischen Einschränkungen. Während GFP eine natürliche "Passform" hat, trifft dies für OspA nicht zu. Als Präzedenz-Fall für ein ungünstig strukturiertes Fremdprotein wurde daher OspA im split-Core-System eingesetzt, und entweder an coreN oder coreC fusioniert. Im Gegensatz zum früheren kontinuierlichen Konstrukt [beschrieben in Nassal et al., 2005] mit breiter Verteilung im Gradienten reicherten sich beide Fusionsproteine distinkt in den Partikel-typischen Fraktionen an. Eine Elektronenmikroskopie-Analyse zeigte eine drastisch verbesserte CLP-Bildungseffizienz verglichen zum alten, kontinuierlichen Konstrukt, sowohl bei Fusion von OspA an coreN wie an coreC.

Dieses Beispiel belegt, daß
(i) das split-core-System die CLP-Präsentation von Fremdproteinen erlaubt, deren Struktur im herkömmlichen kontinuierlichen Core-System mit der CLP-Bildung interferiert,
(ii) im split-Core-System effiziente CLP-Bildung durch Fusion des Fremdproteins sowohl an coreN wie auch coreC möglich ist.

In analoger Weise ließ sich auch Borrelia burgdorferi OspC erfolgreich im erfindungsgemäßen Split-Core-System präsentieren.

### Beispiel 8

### Circumsporozoiten-Protein des Malaria-Erregers Plasmodium falciparum

Ein weiteres unter Vakzine-Gesichtspunkten hochrelevantes Pathogenprotein ist das Circumsporozoiten-Protein (CSP) des Malaria-Erregers *Plasmodium falciparum.* CSP (hier verwendete Form: 319 AS Gesamtlänge) enthält eine ca. 110 AS lange Wiederholungssequenz der Tetrapeptid-Motive NANP/NVDP. Die Struktur von CSP ist nicht bekannt; die C-terminale ca. 50 AS Domäne mit 4 Cys-Resten hat wahrscheinlich (aufgrund Sequenz-Homologie) eine ähnliche Faltung wie Thrombospondin Typ1 repeats. Die repeat-Sequenz ist immunogen, möglicherweise enthalten aber auch die anderen Regionen von CSP wichtige Epitope. Im konventionellen kontinuierlichen Coresystem konnte das Volllängen-CSP nicht in CLP-Form präpariert werden. Dagegen war dies sehr wohl mit dem split-Core-System möglich. Eindeutige Daten liegen derzeit für die Fusion von CSP an coreN vor; bei Koexpression mit coreC (sowohl als 149 wie als 183-Konstrukt) bilden sich effizient partikuläre Strukturen.

Es wurde eine Serie von Konstrukten (sowohl auf Basis von core 1-149 wie 1-183) hergestellt, die entweder nur die repeat-Sequenz, oder trunkiertes CSP ohne Cys-reiche Domäne jeweils im kontinuierlichen oder split-Core-System enthalten; dazu komplettes CSP fusioniert an coreN. Die CLP-Präparationen wurden in einer vergleichenden Immunogenitäts-Studie in Mäusen eingesetzt, die aber noch nicht abgeschlossen werden konnte. Die bisherigen Daten zeigen, dass der im Split-Core-System hergestellte Impfstoff überlegene Eigenschaften aufweist. Dies ist darauf zurückzuführen, dass das komplette CSP ausschließlich bei Anwendung des erfindungsgemäßen Trägersystems die Fähigkeit zur CLP-Bildung aufweist. Bei anderen Systemen entstehen dagegen keine CLPs. Daher dürften mit Hilfe des erfindungsgemäßen Trägersystems hergestellte CSP-Impfstoffe eine überlegene Immunogenität aufweisen und insbesondere neutralisierende Antikörper induzieren.

### Beispiel 9

### Split-Core-CLPs als Träger für Fremdproteine verstärken die B-Zell-Antwort gegen das Fremdprotein

In einer Immunogenitätsstudie in Mäusen werden 5 core-OspA-Konstrukte aus dem split-Core-System vergleichend untersucht mit nicht an Core fusioniertem, lipidiertem OspA (LipOspA); dieses ist die Basis der kommerziellen Lymerix-Vakzine, daher der derzeitige "Goldstandard" für einen Lyme-Krankheits-Impfstoff. Der Lipidanteil in LipOspA (Tris-Palmitoyl-Cystein-(Pam3-Cys)) ist essentiell für dessen relativ hohe Immunogenität, nicht lipidiertes OspA ist nur sehr schwach immunogen (vgl. Nassal et al, 2005). Drei der core-OspA-Konstrukte enthalten Volllängen-OspA (AS 18-273), zwei weitere verkürztes OspA (beginnend bei AS 185, bis AS 273).
6 Gruppen von je 5 BALB/c Mäusen wurden dazu mit je 10 µg Antigen 4-mal immunisiert (Tag 0, 14, 29, 49):
- Gruppe 1:: LipOspA
- Gruppe 2:: Volllängen-OspA (AS 18-273) an coreN, coreC von Core-AS 81 bis 183
- Gruppe 3:: Volllängen-OspA (AS 18-273) an coreN, coreC von Core-AS 81 bis 149
- Gruppe 4:: Verkürztes OspA (AS 185-273) an coreN, coreC von Core-AS 81 bis 183
- Gruppe 5:: Verkürztes OspA (AS 185-273) an coreN, coreC von Core-AS 81 bis 149
- Gruppe 6:: Volllängen-OspA (AS 18-273) an coreC bis Core-AS 183, coreN von AS 1-79

Zur Bestimmung der induzierten Antikörper wurde Blut entnommen an Tag -1 (= 1 Tag vor der ersten Immunisierung; = Präimmunserum), sowie an den Tagen 8, 26, 36 und 57. Mittels ELISA wurden bestimmt: die Kinetik der Bildung OspA-spezifischer Antikörper; der jeweilige Anteil LA2-äquivalenter Antikörper, wobei LA2 ein monoklonaler, bekannt neutralisierender Antikörper ist, der ein komplexes konformationelles Epitop aus diskontinuierlichen AS-Sequenzen *downstream* von AS 185 von OspA erkennt.

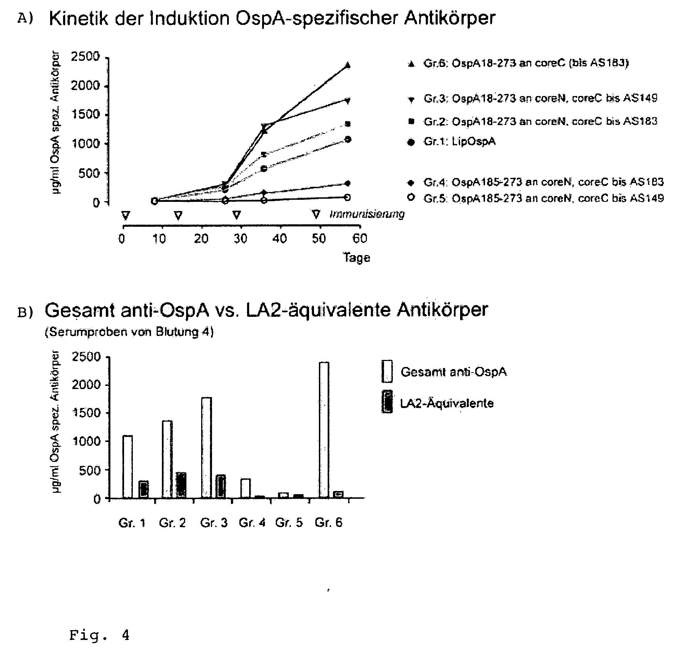

Die Ergebnisse dieses Versuches sind in Fig. 4 detailliert dargestellt.

Fig. 4A zeigt die Kinetik der Induktion von Gesamt-anti-OspA Antikörper (in µg spezifischen Ak pro ml Serum). Bereits nach der zweiten Immunisierung enthalten die Seren aus Mäusen immunisiert mit den Volllängen OspA split-Core Konstrukten nachweisbare und mit den durch LipOspA hervorgerufenen vergleichbare anti-OspA Titer. Nach der 3. und 4. Immunisierung übersteigen die anti-OspA Titer in den split-Core Volllängen-OspA immunisierten Mäusen deutlich diejenigen der mit LipOspA immunisierten Mäuse; besonders hoch sind die Titer nach Immunisierung mit OspA18-273 an coreC (Gruppe 6). Verkürztes OspA185-273 ruft im Kontext von core149 (Gruppe 5) und core183 (Gruppe 4) nur eine wesentlich geringere OspA-spezifische Antwort hervor.

Fig. 4B vergleicht direkt die Gesamt-anti-OspA Ak-Titer mit denjenigen, die gegen das bekannt neutralisierende LA2-Epitop im C-terminalen Bereich von OspA gerichtet sind. Volllängen-OspA an coreN, sowohl im Kontext von core 149 wie core 183 (Gruppe 2 bzw. 3) induzieren höhere anti-LA2-äquivalente Ak-Titer als LipOspA, Volllängen-OspA an coreC deutlich niedrigere. Eine Untersuchung zum jeweiligen Gehalt an *neutralisierenden* Ak (Protektion gegen challenge mit dem *B.burgdorferi* Erreger) wird weiterhihn durchgeführt.

Das Beispiel zeigt, dass split-Core CLPs spezifische Antikörper gegen das präsentierte Volllängen-OspA Fremdprotein induzieren, wobei die Titer höher sind als mit dem etablierten lipidierten LipOspA, bei dem der Lipidanteil wesentlich für die Immunogenität ist. Der unterschiedliche Anteil LA2-äquivalenter Ak nach Immunisierung mit OspA an coreN (ca. 30 %) vs. OspA an coreC (< 5 %) zeigt, dass die Art der Verknüpfung die Art der entstehenden Ak beeinflusst; siehe dazu Beispiel 10.

### Beispiel 10

### Gezielte Induktion regionsspezifischer Antikörper gegen ein Fremdprotein durch alternative Fusion entweder an coreN oder an coreC

Bei Proteinen, deren N- und C-Terminus nicht unmittelbar benachbart sind wie in GFP, führt die Fusion an coreN zu einer anderen Orientierung relativ zur Partikeloberfläche als die Fusion an coreC (coreN: C-Terminus des Fremdprotein "out"; coreC: N-Terminus des Fremdproteins "out"). Es ist zu erwarten, dass bei Verwendung als B-Zell-Vakzine die jeweils am weitesten ins Lösungsmittel ragenden Proteinanteile die stärkste Antikörperantwort hervorrufen. Somit lassen sich unterschiedliche regionsspezifische Antikörper induzieren, indem das Fremdprotein entweder an coreN (C-terminale Seite des Fremdproteins "out") oder an coreC fusioniert wird (N-terminale Seite des Fremdproteins "out").

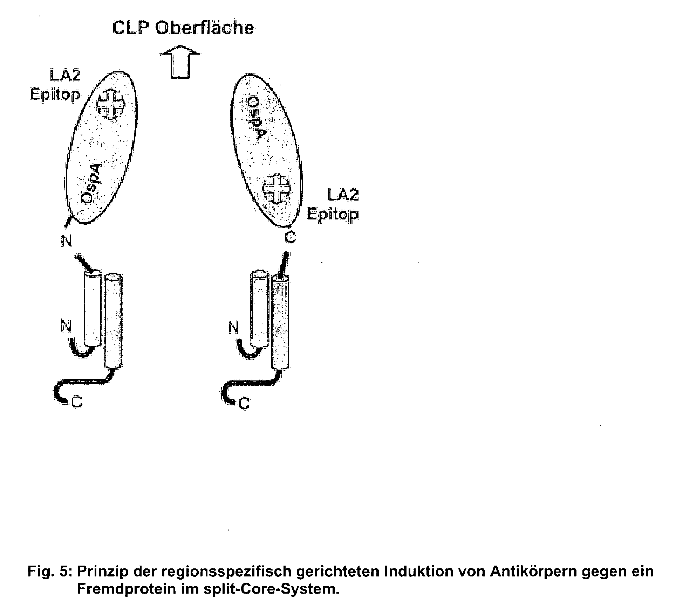

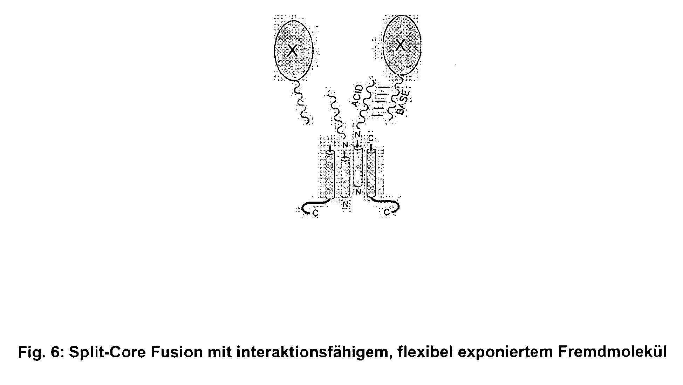

Figur 5 zeigt schematisch, wie bei einem Fremdprotein mit gestreckter Struktur, wie hier OspA, über die Fusion an entweder coreN, oder coreC, bestimmt werden kann, welcher Teil des Moleküls am meisten von der CLP-Oberfläche weg zeigt. Ein gut kartiertes Epitop in OspA ist LA2 im C-terminalen Bereich, das von einem neutralisierenden monoklonalen Antikörper, LA2, erkannt wird. Wie erwartet, induzieren split-Core-OspA CLPs mit OspA an coreN (daher OspA C-Terminus exponiert) eine starke LA2-äquivalente Antwort, CLPs mit OspA an coreC nur eine schwache LA2-äquivalente Antwort. Dafür ist die Antwort gegen andere Bereiche von OspA verstärkt.

Für OspA als Fremdprotein war zu erwarten, dass eine Fusion an coreN das LA2-Epitop im C-terminalen OspA Bereich gut zugänglich macht, eine Fusion an coreC dagegen die bislang immunologisch nicht gut charakterisierte N-terminale OspA Region. Die oben gezeigten Daten bestätigen eindrücklich dieses Konzept (mit OspA an coreC sehr hohe anti-Gesamt-OspA Titer, aber nur geringe LA2-äquivalente Ak-Titer).

Das split-core-System besitzt also eine hohe immunverstärkende Aktivität, sowohl bei Fusion des Fremdproteins an coreN wie an coreC.

Durch Wahl des Verknüpfungsortes (coreN vs. coreC) kann die Art/Regionsspezifität der induzierten Antikörper gesteuert werden. Für optimale Impfergebnisse können Mischungen von Split-Core-Impfstoffen eingesetzt werden, bei denen die heterologe Fremdaminosäuresequenz einmal am Core N und einmal am Core C Teil anfusioniert sind, so dass verschiedene Epitope optimal präsentiert werden.

### Beispiel 11

### Fusionen mit Bedeutung jenseits von Vakzine-Anwendungen

Neben der direkten Bedeutung als immunverstärkender partikulärer Träger für Peptid- und hier besonders Protein-Antigen-Vakzine sind eine Vielzahl weiterer Anwendungen für eine solche Träger-Plattform vorstellbar. Immer wird eine große Anzahl (180 oder 240) präsentierter Moleküle in eine nahe, symmetrische Nachbarschaft gebracht. Handelt es sich um interaktionsfähige Fremdmoleküle (Vakzine stellt einen Spezialfall dar: Interaktion mit Antikörpern), so hat der Partikel mit vielen Kopien des Fremdmoleküls eine drastisch erhöhte Avidität verglichen zum monomeren Fremdmolekül (vgl. natürliche IgM Pentamere; auch MHC-Tetramere in der Immundiagnostik). Damit solche interaktionsfähigen Fremdmoleküle mit ihren Interaktionspartnern reagieren können, müssen sie auf der CLP-Oberfläche zugänglich sein. Dies ist im split-Core-System wesentlich erleichtert gegenüber dem konventionellen kontinuierlichen System.

Eine Reihe von Modell-Fremdsequenzen wurde im split-Core-System exprimiert, alle getesteten Insertionen bildeten CLPs. Für einige wurde die Interaktionsfähigkeit direkt nachgewiesen.

Das split-Arrangement macht auf der CLP-Oberfläche neue Termini für weitere Derivatisierung zugänglich. Die Figur 5 zeigt ein split-Core Dimer mit an coreN fusioniertem ACID-Peptid, welches mit dem komplementären BASE-Peptid interagieren kann. Wurde das BASE-Peptid an ein Molekül X fusioniert, bindet X an die ACID-CLPs. Durch die split-Anordnung ist ACID flexibel; ACID inseriert im kontinuierlichen Core-System interagiert mit BASE-Peptid, da sich aber eine starre coiled-coil Doppelhelix zwischen ACID- und BASE-Peptid ausbildet [O'Shea et al., 1993], wird die Struktur des Core-Trägers instabil, die CLPs zerfallen. In der split-Anordnung sollten die CLPs dagegen stabil bleiben. Diese Koppelung ist generalisierbar: Statt des ACID-Peptides kann ein anderes interaktionsfähiges Molekül A an coreN, oder coreC fusioniert sein. Entsprechende CLPs können dann mit B, einem spezifischen Interaktionspartner von A, interagieren. Solche Interaktionspartner-Paare sind His6 - Ni-NTA, Biotin-Akzeptor-Peptid - Streptavidin; Z33 - Immunglobulin.

Andere Koppelungsmöglichkeiten sind nachfolgend näher erläutert:
a) His6-tag:
   An coreN via Gly2-Linker fusioniert; bildet Partikel, die an Ni2⁺NTA Agarose binden. His-tag im Gegensatz zum kontinuierlichen System frei zugänglich. Vergleichbares Insert (His7) in c/e1 des kontinuierlichen Core-Systems ergab keine nennenswerten Mengen Protein, weil dort der His6-tag Rest sterisch nicht zugänglich ist.
b) Biotin-Akzeptor (BA)-Peptid:
   Biotin-Akzeptor (BA23) ist ein artifizielles 13 AS langes Peptid (GLNDIFEAQKIEWH)), welches durch die Biotin-Ligase BirA aus E. coli biotinyliert wird. Effiziente Biotinylierung erfordert freie Zugänglichkeit; diese ist im split-core-System gegeben, im kontinuierlichen core-System nicht oder nur eingeschränkt. Das split-Core-BA Fusionprotein bildet CLPs und wird in E. coli biotinyliert. An das CLP-präsentierte Biotin kann Avidin/Streptavidin bzw. deren Konjugate gebunden werden.
c) Z 33 Domäne aus Protein A
   S. aureus Protein A bindet Immunglobuline mit hoher Affinität ("Protein-A-Sepharose" für Immunpräzipitation). Protein A besteht aus 5 strukturell ähnlichen lg-Bindedomänen. Z33 ist eine einzige solche Domäne mit modifizierter AS-Sequenz; Gesamtlänge 33 AS). Z33 hat immer noch hohe Affinität zu Ig's (Kd 40 nM). Die Bindung an unterschiedliche Ig's setzt strukturelle Flexibilität voraus, zudem Zugänglichkeit. Die Z33 Sequenz wurde über eine lange Linkersequenz an coreN fusioniert:
   *GGGGSGGGVEDGGGGSGGGGT*-FNMQQQRRFYEALHDPNLNEEQRNAKIKSIREDP.
   Im split-core-System bildeten sich CLPs. Nach Zugabe eines FITC-markierten Immunglobulins und erneuter Sedimentation konnte ein Anteil der Fluoreszenz in den Partikel-typischen Gradientenfraktionen nachgewiesen werden. Dies war nicht der Fall, wenn statt der Z33-Fusion wt-Core-Protein CLPs eingesetzt wurden.
d) ACID Peptid Insert
   Das ACID-Peptid inseriert in das kontinuierliche core-System interagiert mit dem komplementären BASE Peptid ("Peptid-Velcro"= Peptid-Klettverschluss) via coiled-coil Formation der Peptide; [O'Shea et al., 1993] unter massiven Veränderungen der Corestruktur bis hin zum Zerfall; Ursache ist, dass das ACID Peptid alleine eine flexible Struktur annimmt, die keinen "Stress" auf die Corestruktur ausübt. Zugabe des BASE-Peptides führt zur Interaktion mit der ACID-Sequenz, wodurch beide Peptidsequenzen eine starre helicale coiled-coil Konformation annehmen. Wegen der beidseitigen Verknüpfung entsteht Spannung. Ein nur einseitig fixiertes ACID-Peptid sollte spannungsfrei mit zugesetztem BASE-Peptid, oder BASE-Peptid fusioniert an weitere Partner interagieren können. Auf jeden Fall bleibt die Partikelstruktur des ACID-Fusionsproteins im herkömmlichen kontinuierlichen core-System nach Spaltung der C-terminalen Verbindung zum Core-Träger mittels TEV-Protease erhalten.
   Split-Cores mit exponierter ACID-Sequenz können mit Fremdproteinen beladen werden, an die das komplementäre BASE-Peptid gekoppelt ist. Dies ist eine von mehreren Möglichkeiten, split-Core CLPs nachträglich mit Fremdmolekülen der Wahl zu beladen.
   Durch die oben näher erläuterten Kupplungsmechanismen ist es möglich, CLPs mit einem Teil der Kupplung bereitzustellen. Das an den anderen Teil der Kupplung fusionierte Protein kann leicht an die CLPs gekoppelt werden. Auf diese Art können beispielsweise nicht peptidartige Strukturen dem Immunsystem präsentiert werden.
e) Simultane Fusion unterschiedlicher Fremdmoleküle an coreN und coreC
   In den bisherigen Beispielen wurde jeweils eine Fremdsequenz an entweder coreN oder coreC fusioniert. Grundsätzlich können auch *zwei unterschiedliche* Fremdmoleküle simultan an coreN und coreC fusioniert werden; dies könnten bspw. die beiden Untereinheiten eines heterodimeren Fremdproteins sein. Als Modell wurde ein zweigeteiltes GFP benutzt, wovon das eine Segment, die ersten 10 der 11 β-Stränge von GFP enthaltend, an coreN und das zweite Segment, den 11. β-Strang von GFP enthaltend, an coreC fusioniert wurde. Ko-Expression von einem Typ1-Vektor führte zur Entstehung grün fluoreszierender CLPs. Somit hatten sich sowohl die coreN- und coreC-Domänen zu einer assemblierungsfähigen CLP-Struktur zusammengelagert wie auch die beiden GFP-Teile zu einer korrekten 3D-Struktur unter Ausbildung des GFP-Chromophors ergänzt.

Das Beispiel zeigt, dass die simultane Fusion unterschiedlicher Fremdsequenzen an coreN und coreC unter Ausbildung funktioneller Strukturen sowohl des split-Core-Trägers wie auch der beiden unterschiedlichen an coreN und coreC fusionierten Fremdsequenzen möglich ist.

## Patentansprüche

1. Split-Core-Trägersystem, das als getrennte Polypeptide die Core N- und die Core C-Domäne des Core-Proteins von Hepatitis B und wenigstens eine Fremdmolekül, gegen das eine Immunantwort hervorgerufen werden sollen, aufweist, wobei das Fremdmolekül an den C-Terminus der Core N-Domäne oder an den N-Terminus der Core C-Domäne fusioniert ist und das Core-Protein capsid-like-particles binden kann.

2. Split-Core-Trägersystem nach Anspruch 1, **dadurch gekennzeichnet, dass** das Fremdmolekül eine Aminosäuresequenz eines Proteins eines pathogenen Bakteriums ist.

3. Split-Core-Trägersystem nach Anspruch 1, **dadurch gekennzeichnet, dass** das Fremdmolekül eine Aminosäuresequenz eines Proteins eines pathogenen Eukaryonten, insbesondere des Malaria-Erregers Plasmodium falciparum ist.

4. Split-Core-Trägersystem nach Anspruch 1, **dadurch gekennzeichnet, dass** das Fremdmolekül eine Aminosäuresequenz eines Proteins mit eigenem Pathogenitätsbezogenen Potential, insbesondere ein Tumormarkerprotein, ist.

5. Split-Core-Trägersystem nach Anspruch 1, **dadurch gekennzeichnet, dass** das Fremdmolekül ausgewählt ist aus der Gruppe bestehend aus
a) der Sequenz des Biotin-Akzeptor-Peptides
b) der Sequenz des ACID-Peptides
c) der Sequenz der Z33-Domäne abgeleitet von Protein A.

6. Verfahren zur Herstellung eines Trägers für Fremdmoleküle, insbesondere Fremdproteine, gegen die eine Immunantwort induziert werden soll, der einerseits eine Proteinsequenz des Core-Antigens eines Hepatitis B-Virus aufweist und andererseits ein Fremdmolekül, insbesondere eine heterologe Proteinsequenz,
wobei das Fremdmolekül, insbesondere die heterologe Proteinsequenz nach der Position 73 oder vor der Position 94 der Hepatitis B-Coreregion an die Core N oder die Core C Domäne anfusioniert wird und,
wobei die Polypeptidkette des Trägers entweder nach der Domäne Core N oder vor der Domäne Core C unterbrochen wird, **dadurch gekennzeichnet, dass**
entweder die Domäne Core N und das Fremdmolekül, insbesondere die heterologe Proteinsequenz oder die Domäne Core C und das Fremdmolekül, insbesondere die heterologe Proteinsequenz separat als Fusionsproteine exprimiert werden
oder, dass zwischen Core N und Fremdmolekül, insbesondere heterologer Proteinsequenz oder zwischen Fremdmolekül, insbesondere heterologer Proteinsequenz und Core C eine Erkennungssequenz für eine Protease eingefügt wird und der Split-Core-Impfstoff nach der Expression, aber vor der Verwendung mit der Protease in zwei Polypeptide gespalten wird, und,
daß capsid-like particles gebildet werden.

7. Verfahren nach Anspruch 6, **dadurch gekennzeichnet, dass** die heterologe Proteinsequenz nach der Aminosäure 74 oder vor die Aminosäure 85 des Core-Antigens von Hepatitis B anfusioniert wird.

8. Verfahren nach einem der Ansprüche 6-7, **dadurch gekennzeichnet, dass** die heterologe Fremdproteinsequenz wenigstens 40 Aminosäuren lang ist.

9. Verfahren nach Anspruch 7, **dadurch gekennzeichnet, dass** die heterologe Fremdproteinsequenz wenigstens 120 Aminosäuren lang ist.

10. Verfahren nach einem der Ansprüche 6-9, **dadurch gekennzeichnet, dass** zwei separate Polypeptide mit Hilfe eines bicystronischen Vektors exprimiert werden, wobei das eine Polypeptid die Core N-Domäne und das andere Polypeptid die Core C-Domäne beinhaltet und die heterologe Proteinsequenz entweder mit dem C-Terminus der Core N-Domäne oder mit dem N-Terminus der Core C-Domäne fusioniert wird.


SEQUENCE LISTING

<110>  Universitätsklinikum Freiburg

<120>  Split-Core-Partikel für die Präsentation von Fremdmolekülen,
       insbesondere für Impfstoffanwendungen und Verfahren zu deren
       Herstellung

<130>  06018847.1

<160>  9

<170>  PatentIn version 3.3

<210>  1
<211>  17
<212>  PRT
<213>  Artificial

<220>
<223>  Erkennungssequenz für TEV Protease

<400>  1

Gly Gly Gly Gly Thr Glu Asn Leu Tyr Phe Gln Gly Thr Gly Gly Gly
1               5                   10                  15

Gly

<210>  2
<211>  65
<212>  PRT
<213>  Artificial

<220>
<223>  Fusionsprotein mit TEV-protease-Erkennungssequenz

<400>  2

Gly Gly Gly Gly Ser Gly Gly Gly Val Glu Asp Gly Gly Gly Gly Ser
1               5                   10                  15

Gly Gly Gly Gly Thr Ala Gln Leu Glu Lys Glu Leu Gln Ala Leu Glu
            20                  25                  30

Lys Glu Asn Ala Gln Leu Glu Trp Glu Leu Gln Ala Leu Glu Lys Glu
        35                  40                  45

Leu Ala Gln Thr Gly Glu Asn Leu Tyr Phe Gln Gly Thr Gly Gly Gly
    50                  55                  60

Gly
65

<210>  3
<211>  55
<212>  PRT
<213>  Artificial

<220>

<223>  Fusionsprotein mit Z33

<400>  3

Gly Gly Gly Gly Ser Gly Gly Gly Val Glu Asp Gly Gly Gly Gly Ser
1               5                   10                  15

Gly Gly Gly Gly Thr Phe Asn Met Gln Gln Gln Arg Arg Phe Tyr Glu
            20                  25                  30

Ala Leu His Asp Pro Asn Leu Asn Glu Glu Gln Arg Asn Ala Lys Ile
        35                  40                  45

Lys Ser Ile Arg Glu Asp Pro
    50                  55

<210>  4
<211>  14
<212>  PRT
<213>  Artificial

<220>
<223>  Biotin-Akzeptor (BA23)

<400>  4

Gly Leu Asn Asp Ile Phe Glu Ala Gln Lys Ile Glu Trp His
1               5                   10

<210>  5
<211>  6
<212>  PRT
<213>  Artificial

<220>
<223>  Nde-Schnittstelle

<400>  5

Cys Ala Thr Ala Thr Gly
1               5

<210>  6
<211>  6
<212>  PRT
<213>  Artificial

<220>
<223>  Stopcodon upstream Gen

<400>  6

Asn Asn Asn Thr Gly Ala
1               5

<210>  7
<211>  6
<212>  PRT
<213>  Artificial

<220>

<223>  Initionscodon downstream Gen

<400>  7

Asn Asn Ala Thr Gly Ala
1               5

<210>  8
<211>  9
<212>  PRT
<213>  Artificial

<220>
<223>  Typ 1 Konstrukt

<400>  8

Gly Ala Gly Gly Ala Thr Cys Cys Asn
1               5

<210>  9
<211>  10
<212>  PRT
<213>  Artificial

<220>
<223>  Typ 2 Konstrukt

<400>  9

Gly Gly Ala Thr Cys Cys Ala Thr Gly Ala
1               5                   10